# EUROPÄISCHE PATENTSCHRIFT

(11) **EP 1 284 253 B1**
(45) Veröffentlichungstag und Bekanntmachung des Hinweises auf die Patenterteilung: **12.10.2005**
(21) Anmeldenummer: 02013875.6
(22) Anmeldetag: 22.06.2002
(51) Int. Cl.: C07C 17/16

(54) **Verfahren zur Herstellung von Monochlorkohlenwasserstoffen mit hoher Isomerenreinheit**
Process for the preparation of monochloro hydrocarbons with a high isomer purity
Procédé de préparation d'hydrocarbures monochlorés à haute pureté d'isomères

(30) Priorität: 16.08.2001 DE 10140268
(43) Veröffentlichungstag der Anmeldung: 19.02.2003
(73) Patentinhaber: Degussa AG, 40474 Düsseldorf (DE)
(72) Erfinder: Neumann, Manfred, 45770 Marl (DE); Osterholt, Clemens, 46286 Dorsten (DE)

(56) Entgegenhaltungen:
- SANDLER S R: "Cyanuric chloride. A novel laboratory hydrochlorinating reagent for alcohols" JOURNAL OF ORGANIC CHEMISTRY, Bd. 35, Nr. 11, November 1970 (1970-11), Seiten 3967-3968, XP002226615
- DE LUCA L ET AL: "An efficient route to alkyl chlorides from alcohols using the complex TCT/DMF" ORGANIC LETTERS, Bd. 4, Nr. 4, 21. Februar 2002 (2002-02-21), Seiten 553-555, XP002226616

## Beschreibung

Die Erfindung betrifft ein Verfahren zur Herstellung von Monochlorkohlenwasserstoffen, die einen Alkylrest mit 3 bis 20 Kohlenstoffatomen, an den das Chloratom gebunden ist, tragen. Es werden dabei entsprechende Alkohole mit Cyanurchlorid umgesetzt und anschließend das Reaktionsgemisch aufgearbeitet.

Monochlorkohlenwasserstoffe wie Alkylchloride sind unter anderem wertvolle Vorprodukte für Pflanzenschutzmittel und pharmazeutische Erzeugnisse. Sie werden daher oftmals mit einer hohen Isomerenreinheit benötigt.

Bei der Herstellung von Monochlorkohlenwasserstoffen wie Alkylchloriden geht man im Allgemeinen von Olefinen oder Alkoholen aus.

Auch die Herstellung von isomerenreinen Alkylchloriden ist literaturbekannt. So beschreibt S. R. Sandler, J. org. Chem. 35, 3967 (1970) die Umwandlung von primären, sekundären und tertiären Alkoholen in Alkylchloridverbindungen, in dem mit einem Überschuss an Cyanurchlorid unter wasserfreien Bedingungen umgesetzt wird. Weiterhin wird auf Seite 3968 die Umsetzung von 2-Pentanol mit Cyanurchlorid, Zinkchlorid/Chlorwasserstoff, Thionylchlorid/Pyridin und mit Chlorwasserstoff beschrieben. Isomerenreines 2-Chlorpentan wird nur bei der Umsetzung mit Thionylchlorid/Pyridin beziehungsweise mit Cyanurchlorid allein in Ausbeuten von 48 % beziehungsweise 57 % erhalten.

Nachteilig an diesen Verfahren sind insbesondere
a) dass nach der Feststoffabtrennung (Cyanurchlorid, Cyanursäure und Derivate) eine Teilmenge an Cyanurchlorid im Rohmonochlorkohlenwasserstoff verbleibt und es bei der destillativen Aufarbeitung zu einer erheblichen Sublimatbildung kommt,
b) mit zunehmender Kohlenstoffzahl zum Teil sehr lange Reaktionszeiten benötigt werden und
c) bei Verwendung von Thionylchlorid/Pyridin große Mengen an toxischem Pyridin gehandhabt werden müssen und Pyridinhydrochlorid in großen Mengen entsteht.

Aufgabe der vorliegenden Erfindung war es daher, ein einfaches Verfahren bereitzustellen, nach dem isomerenreine Monochlorkohlenwasserstoffe, die einen Alkylrest mit 3 bis 20 Kohlenstoffatomen, an dem das Chloratom gebunden ist und an den weitere zusätzliche Kohlenwasserstoffreste gebunden sein können, enthalten, in hoher Raum/Zeit-Ausbeute hergestellt werden können.

Die Aufgabe wird erfindungsgemäß dadurch gelöst, dass man eine Umsetzung von einem Monoalkohol mit Cyanurchlorid unter Zusatz eines Phasentransferkatalysators (PTC) durchführt und den gebildeten Monochlorkohlenwasserstoff nach einer mechanischen Salzabtrennung und alkalischen Wäsche destillativ reinigt.

Die Umsetzung kann dabei durch das im Folgenden aufgeführte Reaktionsschema schematisch beschrieben werden.

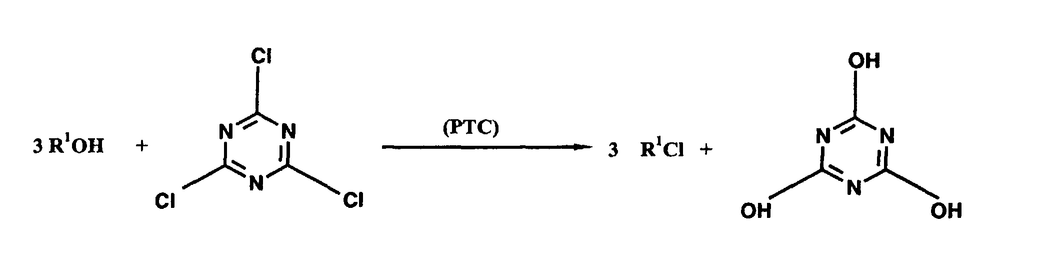

R¹ steht für einen Kohlenwasserstoffrest, der einen primären, sekundären oder tertiären Alkylrest mit 3 bis 20 Kohlenstoffatomen enthält. Der Alkylrest des Kohlenwasserstoffrestes kann unverzweigt oder verzweigt, gesättigt oder ungesättigt sein. An diesem Alkylrest sind zusätzlich 0 bis 3 cycloaliphatische Reste mit 3 bis 8 Kohlenstoffatomen wie zum Beispiel der Cyclopentyl- oder Cyclohexylrest, 0 bis 3 alkylsubstituierte- oder unsubstituierte Aryl-Reste mit 6 bis 16 Kohlenstoffatomen wie zum Beispiel der Phenyl-, der Naphthyl-, ein Toluyl- oder ein Xylyl-Rest, oder 0 bis 3 Aralkyl-Reste mit 7 bis 16 Kohlenstoffatomen wie zum Beispiel der Benzyl- oder der Phenylethyl-Rest gebunden. Vorzugsweise werden sekundäre aliphatische Alkohole mit 5 bis 16, insbesondere 6 bis 16, bevorzugt mit 5 bis 14, insbesondere 6 bis 14, besonders bevorzugt mit 5 bis 12, insbesondere 6 bis 12 Kohlenstoffatomen eingesetzt.

Das Molverhältnis Monoalkohol : Cyanurchlorid beträgt 1 : 3 bis 4 : 1, vorzugsweise 3 : 1,5 bis 3 : 2.

Die Reaktionstemperatur liegt 10 K bis 150 K, vorzugsweise 30 K bis 100 K unterhalb der Siedetemperatur des gebildeten Monochlorkohlenwasserstoffs.

Die Reaktion kann bei einem absoluten Druck von 0,5 bis 10 bar durchgeführt werden. Normaldruck wird der Einfachheit halber bevorzugt.

Die Reaktion kann auch in Gegenwart eines Lösemittels mit einem Siedepunkt, der oberhalb des Siedepunktes des gebildeten Monochlorkohlenwasserstoffs liegt, durchgeführt werden. In diesem Fall kann die Umsetzung des Alkohols mit dem Cyanurchlorid auch oberhalb der entsprechenden Siedetemperatur des gebildeten Monochlorkohlenwasserstoffs erfolgen.

Überraschenderweise wurde gefunden, dass durch Zusatz einer kleinen Menge eines Phasentransferkatalysators die Reaktionszeit deutlich verkürzt werden kann. Beispielsweise wird zur nahezu vollständigen Umsetzung von 6-Undecanol mit Cyanurchlorid bei einer Reaktionstemperatur von 100 °C eine Reaktionszeit von 36 Stunden benötigt (Beispiel 1). Durch Zusatz eines Phasentransferkatalysators ist unter ansonst gleichen Reaktionsbedingungen eine Reaktionszeit von nur 8 Stunden ausreichend (Beispiel 2).

Geeignete Phasentransferkatalysatoren sind zum Beispiel quartäre Ammoniumsalze oder quartäre Phosphoniumsalze, wie Tetraalkylammoniumsalze, vorteilhaft mit C₁ bis C₈ und insbesondere mit C₁ bis C₄-Alkylresten oder Aralkylresten mit 7 bis 16 Kohlenstoffatomen im Aralkylrest, wie zum Beispiel der Benzylrest, oder Alkylarylresten mit 7 bis 16 Kohlenstoffatomen im Alkylarylrest, wie zum Beispiel der Toluyl- oder der Ethylphenyl-Rest. Bei den Salzen kann es sich beispielsweise um Halogenide, Hydrogensulfate oder Sulfate handeln. Vorzugsweise werden Halogenide, insbesondere Chloride eingesetzt. Als Vertreter dieser Stoffklasse seien insbesondere Tetrabutylammoniumchlorid, Benzyltriethylammoniumchlorid und Methyltrioctylammoniumchlorid genannt. Auch Kronenether, insbesondere 18-Krone-6, können als Phasentransferkatalysatoren eingesetzt werden.

Man verwendet die Phasentransferkatalysatoren zweckmäßig in der 0,001- bis 0,2fachen, vorzugsweise 0,005 bis 0,1fachen, besonders bevorzugt 0,01 bis 0,05fachen Gewichtsmenge, bezogen auf das Gewicht des eingesetzten Monoalkohols (0,1 bis 20 Gew.-% bezogen auf den Monoalkohol).

Es sind auch Mischungen von Phasentransferkatalysatoren einsetzbar.

Ein weiterer Vorteil des erfindungsgemäßen Verfahrens liegt in der Aufarbeitung des Reaktionsaustrags. Nach der mechanischen Abtrennung des Feststoffes - vorwiegend ein Gemisch aus Cyanurchlorid, Cyanursäure und Derivaten - beispielsweise durch Filtrieren, Zentrifugieren oder Dekantieren und gegebenenfalls Waschen mit einem Lösemittel, wie zum Beispiel Cyclohexan oder Olefine, verbleibt eine Teilmenge an gelöstem Cyanurchlorid im Filtrat, Zentrifugat oder Dekantat. Als Lösemittel für die Wäsche wird vorzugsweise das bei dem Prozess als Nebenprodukt gebildete Olefin verwendet. Vor der destillativen Aufarbeitung wird das Filtrat einer alkalischen Wäsche unterzogen. Vorzugsweise wird dazu Natronlauge oder Kalilauge eingesetzt. Die Alkalikonzentration der vorzugsweise wässrigen Waschlaugen liegt bei 1 bis 60 Gew.-%, vorzugsweise bei 15 bis 30 Gew.-%. Es wird eine Waschlaugenmenge von 2 bis 80 %, vorzugsweise von 6 bis 12 % bezogen auf den Rohaustrag verwendet.

Anschließend erfolgt zur Reinigung des Monochlorkohlenwasserstoffs eine Destillation, vorzugsweise im Vakuum.

Die bei der Wäsche (Extraktion) anfallende wässrige Phase kann einer speziellen Aufarbeitung für cyanurchloridhaltige Abwässer zugeführt werden.

Die Herstellung der Monochlorkohlenwasserstoffe kann diskontinuierlich oder kontinuierlich erfolgen.

Die folgenden Beispiele sollen die Erfindung weiter erläutern, nicht aber den Anwendungsbereich begrenzen.

### Vergleichsbeispiel 1: Herstellung von 6-Chlorundecan in Abwesenheit eines Phasentransferkatalysators

In einem 1 1-Rührkolben mit Glasflügelrührer und Rückflusskühler werden 250 g (1,445 mol) 6-Undecanol vorgelegt, mit einem Ölbad auf 100 °C eingestellt und 144,4 g (0,783 mol) Cyanurchlorid innerhalb von 2 Stunden zudosiert. Nach einer Gesamtreaktionszeit von 36 Stunden bei 100 °C wird ein ca. 99 %iger 6-Undecanolumsatz erreicht.

Der zweiphasige Reaktionsaustrag (fest/flüssig) wird filtriert und der Feststoff mit Cyclohexan (50 g) gewaschen. Der flüssige Reaktionsaustrag und die Cyclohexan-Waschflüssigkeit werden vereinigt (289,3 g) und mit 67 g einer 8 gew.-%igen Natronlauge extrahiert.

Produktzusammensetzung nach der Natronlaugebehandlung (cyclohexanfrei gerechnet)

| | |
|---|---|
| Undecene | 20 % |
| 6-Chlorundecan | 74 % |
| isomere Chlorundecane | < 0,02 % |
| 6-Undecanol | 1 % |
| Diundecylether | 4 % |
| unbekannter Rest | 1 % |

Bei der destillativen Aufarbeitung wird das Zielprodukt 6-Chlorundecan mit einer Reinheit von > 99 % erhalten. Es wird eine Ausbeute an 6-Chlorundecan von 55 % der Theorie bezogen auf den 6-Undecanoleinsatz erzielt.

### Beispiel 2: Herstellung von 6-Chlorundecan in Anwesenheit eines Phasentransferkatalysators

Die Umsetzung erfolgt analog Beispiel 1. Mit dem 6-Undecanol (250 g) werden jedoch 2,4 g Tetrabutylammoniumchlorid vorgelegt. Nach einer Gesamtlaufzeit von nur 8 Stunden bei 100 °C wird 99 %iger 6-Undecanol-Umsatz erreicht.

Der Anteil an isomeren Chlorundecanen steigt geringfügig auf 0,4 % an. Die Rohproduktzusammensetzung entspricht ansonsten dem Beispiel 1.

### Vergleichsbeispiel 3: Herstellung von 3-Chloroctan

Die Umsetzung erfolgt analog zu Beispiel 1. 81 g (0,522 mol) 3-Octanol werden mit 62,1 g (0,337 mol) Cyanurchlorid in 9 Stunden bei einer Reaktionstemperatur von 70 °C umgesetzt.
Die mit Natronlauge extrahierte flüssige Reaktionsphase hat folgende Zusammensetzung:

| | |
|---|---|
| Octene | 23 % |
| 3-Chloroctan | 69 % |
| isomere Octylchloride | < 0,5 % |
| 3-Octanol | 1,5 % |
| Dioctylether | 5 % |
| unbekannter Rest | < 1 % |

### Vergleichsbeispiel 4: Herstellung von 2-Chlorpentan

Die Umsetzung erfolgt analog zu Beispiel 1: 100 g (1,134 mol) 2-Pentanol werden mit 113 g (0,613 mol) Cyanurchlorid bei einer Reaktionstemperatur von 70 °C innerhalb von 5 Stunden umgesetzt.
Die mit Natronlauge extrahierte flüssige Reaktionsphase hat folgende Zusammensetzung:

| | |
|---|---|
| 2-Methyl-1-buten | 12 % |
| 2-Chlorpentan | 76 % |
| isomere Chlorpentane | -- |
| 2,2'-Oxybispentan | 11 % |
| 2-Pentanol | < 0,3 % |
| unbekannter Rest | < 1 % |

### Vergleichsbeispiel 5: Herstellung von 1-Chlor-1-phenylethan

Die Umsetzung erfolgt analog zu Beispiel 1. 100 g (0,82 mol) 1-Phenylethan-1-ol werden mit 97,6 g (0,529 mol) Cyanurchlorid bei einer Reaktionstemperatur von 90 °C in 14 Stunden umgesetzt. Nach der Phasentrennung, Natronlauge-Behandlung und destillativen Reinigung wird > 99 %iges 1-Chlor-1-phenylethan mit einem Gehalt an isomeren Alkylchloriden von < 0,3 % erhalten.

## Patentansprüche

1. Verfahren zur Herstellung von Monochlorkohlenwasserstoffen mit hoher Isomerenreinheit,
**dadurch gekennzeichnet,**
**dass** ein Monoalkohol, der einen gegebenenfalls ungesättigten Alkylrest mit 3 bis 20 Kohlenstoffatomen, an den weitere Kohlenwasserstoffreste gebunden sein können, enthält, mit Cyanurchlorid umgesetzt, wobei ein Phasentransferkatalysator in einer Konzentration von 0,1 bis 20 Gew.-% bezogen auf den eingesetzten Monoalkohol zugesetzt wird, und der gebildete Monochlorkohlenwasserstoff nach der Salzabtrennung und einer alkalischen Wäsche destillativ gereinigt wird.

2. Verfahren zur Herstellung von Monochlorkohlenwassserstoffen nach Anspruch 1,
**dadurch gekennzeichnet,**
**dass** der Alkylrest 3 bis 20 Kohlenstoffatome enthält und unverzweigt oder verzweigt, gesättigt oder ungesättigt ist, die alkoholische Gruppe an einem primären, sekundären oder tertiären Kohlenstoffatom gebunden ist und der Alkylrest zusätzlich 0 bis 3 cycloaliphatische Reste mit 3 bis 8 Kohlenstoffatomen, 0 bis 3 alkylsubstituierte- oder unsubstituierte Aryl-Reste mit 6 bis 16 Kohlenstoffatomen und 0 bis 3 Arylalkyl-Reste mit 7 bis 16 Kohlenstoffatomen trägt.

3. Verfahren nach Anspruch 1 oder 2,
**dadurch gekennzeichnet,**
**dass** der Monoalkohol ein sekundärer aliphatischer Alkohol ist und 5 bis 16 Kohlenstoffatome enthält.

4. Verfahren nach einem der vorhergehenden Ansprüche,
**dadurch gekennzeichnet,**
**dass** das Molverhältnis Alkohol : Cyanurchlorid 1 : 3 bis 4 : 1 beträgt.

5. Verfahren nach einem der vorhergehenden Ansprüche,
**dadurch gekennzeichnet,**
**dass** die Reaktionstemperatur 10 K bis 150 K unterhalb der Siedetemperatur des gebildeten Monochlorkohlenwasserstoffs liegt.

6. Verfahren nach einem der vorhergehenden Ansprüche,
**dadurch gekennzeichnet,**
**dass** während der Reaktion ein Lösemittel mit einem Siedepunkt, der oberhalb des Siedepunktes des gebildeten Monochlorkohlenwasserstoffs liegt, eingesetzt wird.

7. Verfahren nach einem der vorhergehenden Ansprüche,
**dadurch gekennzeichnet,**
**dass** als Phasentransferkatalysator ein quartäres Ammoniumsalz, ein quartäres Phosphoniumsalz oder ein Kronenether oder eine Mischung dieser Verbindungen eingesetzt wird.

8. Verfahren nach einem der vorhergehenden Ansprüche,
**dadurch gekennzeichnet,**
**dass** als Phasentransferkatalysator ein Tetraalkylammoniumhalogenid eingesetzt wird.

9. Verfahren nach einem der vorhergehenden Ansprüche,
**dadurch gekennzeichnet,**
**dass** das Verfahren diskontinuierlich durchgeführt wird.

10. Verfahren nach einem der vorhergehenden Ansprüche,
**dadurch gekennzeichnet,**
**dass** das Verfahren kontinuierlich durchgeführt wird.

## Claims

1. A process for preparing a monochlorinated hydrocarbon having a high isomeric purity, **characterized in that** a monoalcohol containing a saturated or unsaturated alkyl radical having from 3 to 20 carbon atoms to which further hydrocarbon radicals may be bound is reacted with cyanuric chloride, a phase transfer catalyst being added in a concentration of from 0.1 to 20% by weight based on the monoalcohol used, and the resulting monochlorinated hydrocarbon is purified by distillation after separating off salts and washing with alkali.

2. A process for preparing a monochlorinated hydrocarbon according to claim 1, **characterized in that** the alkyl radical contains from 3 to 20 carbon atoms and is unbranched or branched, saturated or unsaturated, the alcohol group is bound to a primary, secondary or tertiary carbon atom and the alkyl radical additionally bears from 0 to 3 cycloaliphatic radicals having from 3 to 8 carbon atoms, from 0 to 3 alkyl-substituted or unsubstituted aryl radicals having from 6 to 16 carbon atoms and from 0 to 3 arylalkyl radicals having from 7 to 16 carbon atoms.

3. A process according to either of claims 1 and 2, **characterized in that** the monoalcohol is a secondary aliphatic alcohol and contains from 5 to 16 carbon atoms.

4. A process according to any one of the preceding claims, **characterized in that** the molar ratio of alcohol:cyanuric chloride is from 1:3 to 4:1.

5. A process according to any one of the preceding claims, **characterized in that** the reaction temperature is from 10 K to 150 K below the boiling point of the monochlorinated hydrocarbon formed.

6. A process according to any one of the preceding claims, **characterized in that** a solvent having a boiling point above the boiling point of the monochlorinated hydrocarbon formed is used during the reaction.

7. A process according to any one of the preceding claims, **characterized in that** a quaternary ammonium salt, a quaternary phosphonium salt or a crown ether or a mixture of these compounds is used as phase transfer catalyst.

8. A process according to any one of the preceding claims, **characterized in that** a tetraalkylammonium halide is used as phase transfer catalyst.

9. A process according to any one of the preceding claims, **characterized in that** the process is carried out batchwise.

10. A process according to any one of the preceding claims, **characterized in that** the process is carried out continuously.

## Revendications

1. Procédé de préparation d'hydrocarbures monochlorés à haute pureté d'isomères,
**caractérisé en ce qu'**
un monoalcool, qui contient un radical alkyle éventuellement insaturé ayant de 3 à 20 atomes de carbone sur lesquels d'autres radicaux hydrocarbures peuvent être liés, est mis en réaction avec du chlorure cyanurique, en ajoutant un catalyseur par transfert de phases dans une concentration de 0,1 à 20 % en poids rapportée au monoalcool utilisé, et l'hydrocarbure monochloré formé est purifié par distillation après séparation du sel et un lavage alcalin.

2. Procédé de préparation d'hydrocarbures monochlorés selon la revendication 1,
**caractérisé en ce que**
le radical alkyle contient de 3 à 20 atomes de carbone et est non ramifié ou ramifié, saturé ou insaturé, le groupement alcoolisé est lié à un atome de carbone primaire, secondaire ou tertiaire et le radical alkyle porte en plus de 0 à 3 radicaux cycloaliphatiques ayant de 3 à 8 atomes de carbone, de 0 à 3 radicaux aryle substitués par alkyle ou non substitués, ayant de 6 à 16 atomes de carbone, et de 0 à 3 radicaux arylalkyle ayant de 7 à 16 atomes de carbone.

3. Procédé selon la revendication 1 ou 2,
**caractérisé en ce que**
le monoalcool est un alcool secondaire aliphatique et contient de 5 à 16 atomes de carbone.

4. Procédé selon l'une quelconque des revendications précédentes,
**caractérisé en ce que**
le rapport molaire alcool / chlorure cyanurique est de 1 / 3 à 4 / 1.

5. Procédé selon l'une quelconque des revendications précédentes,
**caractérisé en ce que**
la température de réaction se situe à 10 K à 150 K en dessous de la température d'ébullition de l'hydrocarbure monochloré formé.

6. Procédé selon l'une quelconque des revendications précédentes,
**caractérisé en ce que**
pendant la réaction, on utilise un solvant ayant un point d'ébullition situé au-dessus du point d'ébullition de l'hydrocarbure monochloré formé.

7. Procédé selon l'une quelconque des revendications précédentes,
**caractérisé en ce que**
comme catalyseur par transfert de phases, on utilise un sel d'ammonium quaternaire, un sel de phosphonium quaternaire ou un éther couronne ou un mélange de ces composés.

8. Procédé selon l'une quelconque des revendications précédentes,
**caractérisé en ce que**
comme catalyseur par transfert de phases, on utilise un halogénure de tétraalkylammonium.

9. Procédé selon l'une quelconque des revendications précédentes,
**caractérisé en ce que**
le procédé est réalisé de façon discontinue.

10. Procédé selon l'une quelconque des revendications précédentes,
**caractérisé en ce que**
le procédé est réalisé en continu.
